# EUROPÄISCHE PATENTSCHRIFT

(11) **EP 3 166 691 B1**
(45) Veröffentlichungstag und Bekanntmachung des Hinweises auf die Patenterteilung: **16.01.2019**
(21) Anmeldenummer: 15726141.3
(22) Anmeldetag: 01.06.2015
(51) Int. Cl.: A61Q 11/00, A61K 8/81

(54) **MUND- UND ZAHNPFLEGE- UND REINIGUNGSMITTEL MIT WIRKSTOFFDEPOSITION**
ORAL AND DENTAL CARE AND CLEANING PRODUCTS WITH ACTIVE AGENT DEPOSITION
AGENTS D'HYGIÈNE BUCCO-DENTAIRE ET DE NETTOYAGE COMPORTANT UN DÉPÔT DE SUBSTANCE ACTIVE

(30) Priorität: 07.07.2014 DE 102014213158
(43) Veröffentlichungstag der Anmeldung: 17.05.2017
(73) Patentinhaber: Henkel AG & Co. KGaA, 40589 Düsseldorf (DE)
(72) Erfinder: MIEHLICH, Kristin, 42119 Wuppertal (DE); DUSCHEK, Nicole, 40595 Düsseldorf (DE); ARIANS, Daniela, 45239 Essen (DE)
(86) Internationale Anmeldenummer: PCT/EP2015/062109
(87) Internationale Veröffentlichungsnummer: WO 2016/005106

(56) Entgegenhaltungen:
- WO-A1-2005/034897
- WO-A1-2005/077326
- WO-A2-2010/054494
- US-A- 4 364 924
- US-A- 4 364 927
- US-A1- 2006 024 246
- US-A1- 2006 088 482
- US-A1- 2007 140 992
- DATABASE GNPD [Online] MINTEL; Oktober 2009 (2009-10), "Brush on Tooth Veneer", XP002742618, Database accession no. 1195499
- DATABASE GNPD [Online] MINTEL; März 2011 (2011-03), "Teeth Whitener", XP002742619, Database accession no. 1510229

## Beschreibung

Die Erfindung betrifft Mund- und Zahnpflegemittel mit einer Wirkstoffkombination zur schonenden und effektiven Reinigung der Zähne.

Viele Menschen wünschen sich weiße Zähne und empfinden dunkle oder verfärbte Zähne als kosmetisch inakzeptabel. Die Aufrechterhaltung der natürlichen Zahnfarbe ist jedoch trotz regelmäßiger Dentalhygiene nicht immer erfolgreich. Ernährungsgewohnheiten oder Rauchen können zu Zahnverfärbungen führen. Ebenso führt die Besiedlung der Zahnoberfläche mit Bakterien (Plaque) zu Verfärbungen.

Eine Reihe von technischen Lösungen zur Entfernung oder Aufhellung von Zähnen wurde entwickelt. Zur Aufhellung/Bleichen kommt vor allem Peroxid zum Einsatz. In professionellen Bleichprodukten wird Peroxid in hohen Konzentrationen eingesetzt, während der Einsatz in Kosmetikprodukten zur Mund- und Zahnpflege auf 0,1% Peroxid beschränkt ist. Peroxid in dieser Konzentration hat nur eine eingeschränkte aufhellende Wirkung und kann Verfärbungen der Zähne oft nicht im gewünschten Maß beseitigen.

Eine weitere Möglichkeit zur Aufhellung der Zähne besteht in der effektiven Entfernung von Zahnbelägen, welche die Zähne dunkler aussehen lassen. Diese Methode der Zahnaufhellung wird auch als "Natural Whitening" beschrieben. Eine hohe Reinigungsleistung wird am besten erreicht durch Putzkörper, zum Beispiel Silica, Alumina oder Calciumcarbonat in Kombination mit einem Tensid. Leider weisen Zahncremes mit einem effektiven System aus einem oder mehreren Putzkörpern häufig auch eine hohe Abrasivität auf, führen also zu einem gewissen, wenn auch sehr geringem Abrieb der Zahnoberfläche. Dies kann sich insbesondere dann nachteilig auswirken, wenn das Enamel eines Zahnes ohnehin dünn ist, wie dies bei Personen mit empfindlichen Zähnen der Fall ist. Oft treten bei Personen mit empfindlichen Zähnen auch freiliegende Zahnhälse auf, also Partien des Zahnes in unmittelbarer Nähe zum Zahnfleisch, an denen kein Enamel als Schutzschicht vorhanden ist und das darunter liegende Dentin frei liegt.

Zudem bildet sich sofort nach dem Zähneputzen auf den Zahnmaterial eine Eiweißschicht (Pellicle) auf der sich der Plaque aufbaut bzw. Verfärbungen anlagern.

Es besteht daher ein Bedarf an Zahnpasten, die eine effektive Reinigung und Aufhellung bewirken aber dabei gleichzeitig einen Langzeiteffekt bewirken, d.h. die Bildung neuer Plaque und damit neuer Verfärbungen möglichst lange verhindern bzw. verringern. Gleichzeitig sollte eine möglichst lange antibakterielle Wirkung erzielt werden. Zudem sollten weitere Zusatznutzen inkorporiert werden können, beispielsweise die Verbesserung des optischen Erscheinungsbildes der Zähne durch Bleichen oder durch Fixierung blauer Farbstoffe und/oder Pigmente auf der Zahnoberfläche oder Verlängerung der Kariesprophylaxe oder des Zahnfleischschutzes.

Die Fixierung von Farbstoffen auf der Zahnoberfläche mittels Polymeren zur Erzielung eines weißeren Erscheinungsbildes ist z.B. aus der WO2005/077326 A1 bekannt. Diese Schrift beschreibt ein System aus filmformenden Polymer mit Farbstoff zur temporären Einfärbung von Zähnen. Das dort beschriebene Produkt muß direkt auf die Zähne appliziert werden und dort verbleiben. Die Anwendung ist für den Konsumenten umständlich und zeitaufwändig.
Die EP 1 935 395 A1 beschreibt den Effekt im b*-Wert für eine Kombination aus nicht filmbildenden Polymeren und blauen Pigmenten. Die Pigmente sollen auf der Zahnoberfläche verbleiben und dort mit der gelblichen Grundfärbung einen weißeren Gesamteindruck vermitteln. Pigmente weisen jedoch den Nachteil auf, daß sie nicht in jedem Produkt stabil formulierbar sind. Zudem besteht die Gefahr, daß Textilien, Keramik und andere Materialien durch Pigmente eingefärbt werden.

Die WO 2010/054494 offenbart eine zweiteilige Mundpflegezusammensetzung wobei eine Zusammensetzung eine Phosphatquelle und die andere Zusammensetzung eine Calciumquelle und eine der beiden Zusammensetzungen zusätzlich filmbildendes Polymer enthält.

Produkte, die Acrylates/Ammonium Acrylate Coloymer enthalten, sind im Markt erhältlich, beispielsweise der "brush on tooth veener" von Church&Dwight (Irland) oder der "teeth whitener" von Sofibel (Frankreich).

Die US 2006/024246 offenbart Mundpflegezusammensetzungen, enthaltend filmbildende Acrylatpolymere und oralaktive Wirkstoffe.

Die US 4,364,927 und die US 4,364,924 offenbaren filmbildende Polymere zur Verhinderung der Adhäsion von Plaque an Zähnen. Beispielhafte Mittel enthalten z.B. FD&C Blue 1 als Farbstoff.

Die WO 2005/034897 offenbart Parfumzusammensetzungen, enthaltend filmbildende Polymere.

In der WO 2005/077326 werden Zahnlacke offenbart, enthaltend eine filmbildende Kombination aus natürlichen Harzen und Farbstoff.

Die US 2007 140 992 offenbart Mundpflegemittel enthaltend ein Hydrokolloid und ein essentielles Öl, enthaltend u.a. Thymol.

Die US 2006 088 482 offenbart Mund- und Zahnpflegemittel enthaltend kationische antibakterielle Verbindungen, Diphosphonoazacycloalkane und Bindemittel.

Die Aufgabe der vorliegenden Erfindung bestand darin, Zubereitungen zur Mund- und Zahnpflege und -reinigung bereitzustellen, die eine effektive Reinigung, Plaquereduktion und Aufhellung bewirken und dabei neue Verfärbungen möglichst lange verhindern bzw. verringern. Auch die Antibakterielle Wirkung der erfindungsgemäßen Mittel sollte länger anhaltend sein als die bekannter Mittel. Überraschenderweise wurde nun gefunden, daß sich bestimmte Polymere besonders dazu eignen, die verschiedensten Aktivstoffe an die Zahnoberfläche und das Zahnfleisch zu binden. In ihrer Wirkung sind sie einerseits den aus dem Stand der Technik bekannten Polymeren überlegen, anderseits sind sie universeller einsetzbar, da sie neben Farbstoffen eine Vielzahl anderer Substanzen fixieren können.

Gegenstand der Erfindung sind daher Mund- und Zahnpflege- und -reinigungsmittel, enthaltend - bezogen auf ihr Gewicht -
a) 0,1 bis 5 Gew.-% Copolymer(e), umfassend mindestens eine Struktureinheit der Formel (A1) und/oder mindestens eine Struktureinheit der Formel (A2) 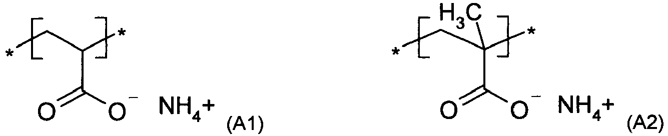
mit der Maßgabe, daß sie - bezogen auf ihr Gewicht - 0,15 - 4 Gew.-%, vorzugsweise 0,2 - 3 Gew.-%, besonders bevorzugt 0,25 - 2,75 Gew.-%, außerordentlich bevorzugt 0,5 - 2,5 Gew.-% und insbesondere 1 bis 2 Gew.-% Copolymer(e) aus der Gruppe der Copolymere, die jeweils ausschließlich aus folgenden Monomeren aufgebaut sind:
- Ammoniumacrylat und Acrylsäure;
- Ammoniumacrylat und Methacrylsäure;
- Ammoniumacrylat und Acrylsäureethylester;
- Ammoniumacrylat und Methacrylsäureethylester;
- Ammoniummethacrylat und Acrylsäure;
- Ammoniummethacrylat und Methacrylsäure;
- Ammoniummethacrylat und Acrylsäureethylester;
- Ammoniummethacrylat und Methacrylsäureethylester;
- Ammoniumacrylat und Ammoniummethacrylat und Acrylsäure;
- Ammoniumacrylat und Ammoniummethacrylat und Methacrylsäure;
- Ammoniumacrylat und Ammoniummethacrylat und Acrylsäureethylester;
- Ammoniumacrylat und Ammoniummethacrylat und Methacrylsäureethylester;
- Ammoniumacrylat und Acrylsäure und Methacrylsäure;
- Ammoniumacrylat und Acrylsäure und Acrylsäureethylester;
- Ammoniumacrylat und Acrylsäure und Methacrylsäureethylester;
- Ammoniumacrylat und Methacrylsäure und Acrylsäureethylester;
- Ammoniumacrylat und Methacrylsäure und Methacrylsäureethylester;
- Ammoniumacrylat und Acrylsäureethylester und Methacrylsäureethylester;
- Ammoniummethacrylat und Acrylsäure und Methacrylsäure;
- Ammoniummethacrylat und Acrylsäure und Acrylsäureethylester;
- Ammoniummethacrylat und Acrylsäure und Methacrylsäureethylester;
- Ammoniummethacrylat und Methacrylsäure und Acrylsäureethylester;
- Ammoniummethacrylat und Methacrylsäure und Methacrylsäureethylester;
- Ammoniummethacrylat und Acrylsäureethylester und Methacrylsäureethylester;
- Ammoniumacrylat und Ammoniummethacrylat und Acrylsäure und Methacrylsäure;
- Ammoniumacrylat und Ammoniummethacrylat und Acrylsäure und Acrylsäureethylester;
- Ammoniumacrylat und Ammoniummethacrylat und Acrylsäure und Methacrylsäureethylester;
- Methacrylsäuremethylester;
- Ammoniumacrylat und Ammoniummethacrylat und Methacrylsäure und Acrylsäureethylester;
- Ammoniumacrylat und Ammoniummethacrylat und Methacrylsäure und Methacrylsäureethylester;
- Ammoniumacrylat und Ammoniummethacrylat und Acrylsäureethylester und Methacrylsäureethylester;
enthalten.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Zusammensetzungen bezogen auf ihr Gewicht 0,1 bis 5 Gew.-% Copolymer(e), umfassend mindestens eine Struktureinheit der Formel (A1) und/oder mindestens eine Struktureinheit der Formel (A2), mit der Maßgabe, daß
sie - bezogen auf ihr Gewicht - 0,15 - 4 Gew.-%, vorzugsweise 0,2 - 3 Gew.-%, besonders bevorzugt 0,25 - 2,75 Gew.-%, außerordentlich bevorzugt 0,5 - 2,5 Gew.-% und insbesondere 1 bis 2 Gew.-% Copolymer(e) aus der Gruppe der Copolymere, die jeweils ausschließlich aus folgenden Monomeren aufgebaut sind:
- Ammoniumacrylat und Acrylsäure;
- Ammoniumacrylat und Methacrylsäure;
- Ammoniumacrylat und Acrylsäureethylester;
- Ammoniumacrylat und Methacrylsäureethylester;
- Ammoniummethacrylat und Acrylsäure;
- Ammoniummethacrylat und Methacrylsäure;
- Ammoniummethacrylat und Acrylsäureethylester;
- Ammoniummethacrylat und Methacrylsäureethylester;
- Ammoniumacrylat und Ammoniummethacrylat und Acrylsäure;
- Ammoniumacrylat und Ammoniummethacrylat und Methacrylsäure;
- Ammoniumacrylat und Ammoniummethacrylat und Acrylsäureethylester;
- Ammoniumacrylat und Ammoniummethacrylat und Methacrylsäureethylester;
- Ammoniumacrylat und Acrylsäure und Methacrylsäure;
- Ammoniumacrylat und Acrylsäure und Acrylsäureethylester;
- Ammoniumacrylat und Acrylsäure und Methacrylsäureethylester;
- Ammoniumacrylat und Methacrylsäure und Acrylsäureethylester;
- Ammoniumacrylat und Methacrylsäure und Methacrylsäureethylester;
- Ammoniumacrylat und Acrylsäureethylester und Methacrylsäureethylester;
- Ammoniummethacrylat und Acrylsäure und Methacrylsäure;
- Ammoniummethacrylat und Acrylsäure und Acrylsäureethylester;
- Ammoniummethacrylat und Acrylsäure und Methacrylsäureethylester;
- Ammoniummethacrylat und Methacrylsäure und Acrylsäureethylester;
- Ammoniummethacrylat und Methacrylsäure und Methacrylsäureethylester;
- Ammoniummethacrylat und Acrylsäureethylester und Methacrylsäureethylester;
- Ammoniumacrylat und Ammoniummethacrylat und Acrylsäure und Methacrylsäure;
- Ammoniumacrylat und Ammoniummethacrylat und Acrylsäure und Acrylsäureethylester;
- Ammoniumacrylat und Ammoniummethacrylat und Acrylsäure und Methacrylsäureethylester;
- Methacrylsäuremethylester;
- Ammoniumacrylat und Ammoniummethacrylat und Methacrylsäure und Acrylsäureethylester;
- Ammoniumacrylat und Ammoniummethacrylat und Methacrylsäure und Methacrylsäureethylester;
- Ammoniumacrylat und Ammoniummethacrylat und Acrylsäureethylester und Methacrylsäureethylester;
enthalten.
- Ammoniumacrylat und Ammoniummethacrylat und Acrylsäuremethylester;
- Ammoniumacrylat und Ammoniummethacrylat und Methacrylsäuremethylester;
- Ammoniumacrylat und Ammoniummethacrylat und Acrylsäureethylester;
- Ammoniumacrylat und Ammoniummethacrylat und Methacrylsäureethylester;
- Ammoniumacrylat und Ammoniummethacrylat und Acrylsäurepropylester;
- Ammoniumacrylat und Ammoniummethacrylat und Methacrylsäurepropylester;
- Ammoniumacrylat und Ammoniummethacrylat und Acrylsäureisopropylester;
- Ammoniumacrylat und Ammoniummethacrylat und Methacrylsäureisopropylester;
- Ammoniumacrylat und Acrylsäure und Methacrylsäure;
- Ammoniumacrylat und Acrylsäure und Acrylsäuremethylester:
- Ammoniumacrylat und Acrylsäure und Methacrylsäuremethylester;
- Ammoniumacrylat und Acrylsäure und Acrylsäureethylester;
- Ammoniumacrylat und Acrylsäure und Methacrylsäureethylester;
- Ammoniumacrylat und Methacrylsäure und Acrylsäuremethylester;
- Ammoniumacrylat und Methacrylsäure und Methacrylsäuremethylester;
- Ammoniumacrylat und Methacrylsäure und Acrylsäureethylester;
- Ammoniumacrylat und Methacrylsäure und Methacrylsäureethylester;
- Ammoniumacrylat und Acrylsäuremethylester und Methacrylsäuremethylester;
- Ammoniumacrylat und Acrylsäuremethylester und Acrylsäureethylester;
- Ammoniumacrylat und Acrylsäuremethylester und Methacrylsäureethylester;
- Ammoniumacrylat und Methacrylsäuremethylester und Acrylsäureethylester;
- Ammoniumacrylat und Methacrylsäuremethylester und Methacrylsäureethylester;
- Ammoniumacrylat und Acrylsäureethylester und Methacrylsäureethylester;
- Ammoniummethacrylat und Acrylsäure und Methacrylsäure;
- Ammoniummethacrylat und Acrylsäure und Acrylsäuremethylester:
- Ammoniummethacrylat und Acrylsäure und Methacrylsäuremethylester;
- Ammoniummethacrylat und Acrylsäure und Acrylsäureethylester;
- Ammoniummethacrylat und Acrylsäure und Methacrylsäureethylester;
- Ammoniummethacrylat und Methacrylsäure und Acrylsäuremethylester;
- Ammoniummethacrylat und Methacrylsäure und Methacrylsäuremethylester;
- Ammoniummethacrylat und Methacrylsäure und Acrylsäureethylester;
- Ammoniummethacrylat und Methacrylsäure und Methacrylsäureethylester;
- Ammoniummethacrylat und Acrylsäuremethylester und Methacrylsäuremethylester;
- Ammoniummethacrylat und Acrylsäuremethylester und Acrylsäureethylester;
- Ammoniummethacrylat und Acrylsäuremethylester und Methacrylsäureethylester;
- Ammoniummethacrylat und Methacrylsäuremethylester und Acrylsäureethylester;
- Ammoniummethacrylat und Methacrylsäuremethylester und Methacrylsäureethylester;
- Ammoniummethacrylat und Acrylsäureethylester und Methacrylsäureethylester;
- Ammoniumacrylat und Ammoniummethacrylat und Acrylsäure und Methacrylsäure;
- Ammoniumacrylat und Ammoniummethacrylat und Acrylsäure und Acrylsäuremethylester:
- Ammoniumacrylat und Ammoniummethacrylat und Acrylsäure und Methacrylsäuremethylester;
- Ammoniumacrylat und Ammoniummethacrylat und Acrylsäure und Acrylsäureethylester;
- Ammoniumacrylat und Ammoniummethacrylat und Acrylsäure und Methacrylsäureethylester;
- Ammoniumacrylat und Ammoniummethacrylat und Methacrylsäure und Acrylsäuremethylester;
- Ammoniumacrylat und Ammoniummethacrylat und Methacrylsäure und Methacrylsäuremethylester;
- Ammoniumacrylat und Ammoniummethacrylat und Methacrylsäure und Acrylsäureethylester;
- Ammoniumacrylat und Ammoniummethacrylat und Methacrylsäure und Methacrylsäureethylester;
- Ammoniumacrylat und Ammoniummethacrylatund Acrylsäuremethylester und Methacrylsäuremethylester;
- Ammoniumacrylat und Ammoniummethacrylat und Acrylsäuremethylester und Acrylsäureethylester;
- Ammoniumacrylat und Ammoniummethacrylat und Acrylsäuremethylester und Methacrylsäureethylester;
- Ammoniumacrylat und Ammoniummethacrylat und Methacrylsäuremethylester und Acrylsäureethylester;
- Ammoniumacrylat und Ammoniummethacrylat und Methacrylsäuremethylester und Methacrylsäureethylester;
- Ammoniumacrylat und Ammoniummethacrylat und Acrylsäureethylester und Methacrylsäureethylester;

Äußerst bevorzugte erfindungsgemäße Mund- und Zahnpflege- und -reinigungsmittel sind dabei dadurch gekennzeichnet, daß sie - bezogen auf ihr Gewicht - 0,15 - 4 Gew.-%, vorzugsweise 0,2 - 3 Gew.-%, besonders bevorzugt 0,25 - 2,75 Gew.-%, außerordentlich bevorzugt 0,5 - 2,5 Gew.-% und insbesondere 1 bis 2 Gew.-% Copolymer(e) aus der Gruppe der Copolymere, die jeweils ausschließlich aus folgenden Monomeren aufgebaut sind:
- Ammoniumacrylat und Acrylsäure;
- Ammoniumacrylat und Methacrylsäure;
- Ammoniumacrylat und Acrylsäureethylester;
- Ammoniumacrylat und Methacrylsäureethylester;
- Ammoniummethacrylat und Acrylsäure;
- Ammoniummethacrylat und Methacrylsäure;

Gemäß obiger Formeln und allen folgenden Formeln steht eine chemische Bindung, die mit dem Symbol * gekennzeichnet ist, für eine freie Valenz des entsprechenden Strukturfragments.

Es hat sich gezeigt, daß Copolymere a) eines bestimmten Molmassenbereiches in den erfindungsgemäßen Mitteln besonders wirksam sind. Besonders bevorzugte erfindungsgemäße Mund- und Zahnpflege- und reinigungsmittel sind daher dadurch gekennzeichnet, daß das/die Copolymer(e), umfassend mindestens eine Struktureinheit der Formel (A1) und mindestens eine Struktureinheit der Formel (A2), Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

Die erfindungsgemäßen Zusammensetzungen eignen sich aufgrund des Einsatzes des/der genannten Copolymers/Copolymere hervorragend, um weitere Zusatznutzen in die Mittel zu inkorporieren, beispielsweise die Verbesserung des optischen Erscheinungsbildes der Zähne durch Bleichen oder durch Fixierung blauer Farbstoffe und/oder Pigmente auf der Zahnoberfläche oder Verlängerung der Kariesprophylaxe oder des Zahnfleischschutzes.

Aus diesem Grunde ist es bevorzugt, solche Stoffe in die erfindungsgemäßen Mittel zu inkoporieren, die sich mit Hilfe der erfindungsgemäß eingesetzten Copolymere besonders nachhaltig auf der Zahnoberfläche und im Mundraum fixieren lassen und durch den Gehalt der Mittel an diesen Polymeren deutlich wirkverstärkt gegenüber Mitteln, die diese Polymere nicht enthalten, sind. Danz besonders bevorzugte fixierbare Inhaltsstoffe sind nachstehend beschrieben. Besonders bevorzugte erfindungsgemäße Mund- und Zahnpflege- und reinigungsmittel sind dadurch gekennzeichnet, daß sie mindestens einen weiteren Inhaltsstoff aus der Gruppe
- der blauen Farbstoffe und/oder
- der Verbindungen der Formel (PP-I) 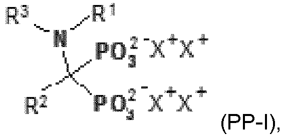 worin
   R¹ für Wasserstoff oder eine lineare oder verzweigte Alkylgruppe mit 1 bis 6 Kohlenstoffatomen steht,
   R² und R³, jeweils unabhängig voneinander, für eine lineare oder verzweigte Alkylgruppe mit 1 bis 6 Kohlenstoffatomen stehen oder R² und R³ eine cyclische Gruppe mit 4 bis 8 Kohlenstoffatomen und gegebenenfalls einem Heteroatom, ausgewählt aus der Gruppe von N, O und S, bilden, und
   X⁺ unabhängig voneinander für H⁺, Li⁺, Na⁺, K⁺, NH₄⁺, ½Mg²⁺, ½Ca²⁺, ½Mn²⁺, ½Zn²⁺, ⅓Al³⁺, ¼Zr⁴⁺ steht und/oder
- der Isopropylmethylphenole und/oder
- Pentanatriumtriphosphat und/oder
- der Zinksalze
enthalten.

Als einen bevorzugten Inhaltsstoff können die erfindungsgemäßen Mittel zusätzlich zu dem/den Copolymer(en) mindestens einen Blaufarbstoff enthalten.

Bevorzugte erfindungsgemäße Zusammensetzungen enthalten 0,0001 bis 1 Gew.-% mindestens eines blauen Farbstoffes.

Die Kombination von auf den Zähnen substantivem Polymer und blauem Farbstoff modifiziert die Oberfläche der Zähne derart, daß das von den Zähnen reflektierte Licht im L*a*b* Farbraum einen geringeren b*-Wert aufweist. Unbehandelte Zähne haben in der Regel einen positiven b*-Wert, erscheinen also mit in einem gelblichen Ton. Eine Reduzierung des b*-Wertes durch das Mundwasser mit der beschriebenen Kombination verändert die Zahnfarbe in Richtung eines neutraleren Farbtons, was das Auge als weißer wahrnimmt. Erfindungsgemäß werden Farbstoffe eingesetzt, d.h. Verbindungen, die im Anwendungsmedium löslich sind. Die Nachteile von Pigmenten (Einsatz einer gegen Sedimentation zu stabilisierenden Suspension, Problematik der schwer zu entfernenden Anfärbung) werden dadurch vermieden. Überraschenderweise führt auch der Einsatz löslicher Farbstoffe zu einem deutlichen und lang anhaltenden Weißen der Zähne.

Erfindungsgemäß geeignet sind alle Blaufarbstoffe, unabhängig von ihrer chemischen Struktur. Besonders bewährt haben sich im Rahmen der vorliegenden Erfindung Triphenylmethanfarbstoffe, darunter insbesondere die verschiedenen Patentblau-Farbstoffe Patentblau VF (acid blue 1), Patentblau AF (acid blue 7) und Patentblau V (acid blue, C.I. 42051) sowie Brillantblau FCF (Patentblau AE, acid blue 9, C.I. 42090) sowie Indigofarbstoffe, insbesondere Indigotin (C.I. 73015). Unabhängig von der Art des Blaufarbstoffes bzw. der Blaufarbstoffe sind erfindungsgemäße Zusammensetzungen bevorzugt, die bezogen auf ihr Gewicht 0,0002 bis 0,5 Gew.-%, vorzugsweise 0,0005 bis 0,25 Gew.-%, besonders bevorzugt 0,001 bis 0,1 Gew.-% und insbesondere 0,001 bis 0,05 Gew.-% mindestens eines blauen Farbstoffes enthalten.

Bevorzugte erfindungsgemäße Zusammensetzungen sind dadurch gekennzeichnet, daß sie, bezogen auf ihr Gewicht 0,0002 bis 0,5 Gew.-%, vorzugsweise 0,0005 bis 0,25 Gew.-%, besonders bevorzugt 0,001 bis 0,1 Gew.-% und insbesondere 0,002 bis 0,05 Gew.-% *4-[4,4'-Bis-diethylamino-α-hydroxy-benzhydryl]-6-hydroxy-benzol-1,3-disulfonsäure* (Patentblau V) 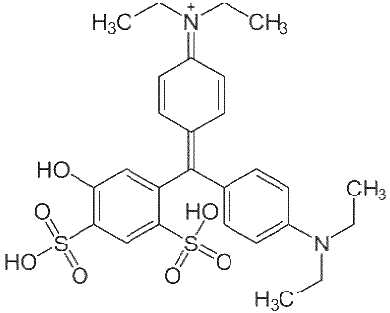 enthalten.

Weiter bevorzugte erfindungsgemäße Zusammensetzungen sind dadurch gekennzeichnet, daß sie, bezogen auf ihr Gewicht 0,0002 bis 0,5 Gew.-%, vorzugsweise 0,0005 bis 0,25 Gew.-%, besonders bevorzugt 0,001 bis 0,1 Gew.-% und insbesondere 0,002 bis 0,05 Gew.-% Indigotin-5,5'-disulfonsäure Dinatriumsalz 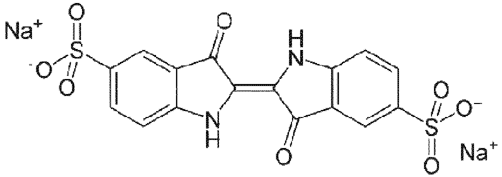 enthalten.

Insbesondere bevorzugte erfindungsgemäße Mund- und Zahnpflege- und reinigungsmittel sind dadurch gekennzeichnet, daß sie, bezogen auf ihr Gewicht 0,0002 bis 0,5 Gew.-%, vorzugsweise 0,0005 bis 0,25 Gew.-%, besonders bevorzugt 0,001 bis 0,1 Gew.-% und insbesondere 0,002 bis 0,05 Gew.-% Benzenemethanaminium,N-ethyl-N-[4-[[4-[ethyl[(3-sulfophenyl)methyl]amino]phenyl](2-sulfophenyl)methylene]-2,5-cyclohexadien-1-ylidene]-3-sulfo-,inner salt, sodium salt (Brillantblau FCF) 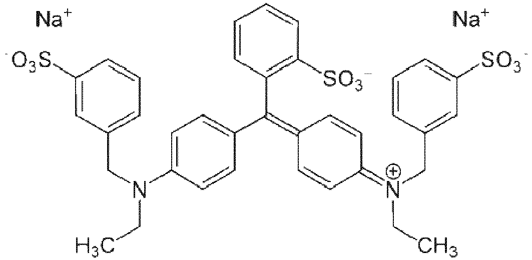 enthalten.

Als einen bevorzugten Inhaltsstoff können die erfindungsgemäßen Mittel zusätzlich zu dem/den Copolymer(en) mindestens eine Verbindung der Formel (PP-I) enthalten.

Erfindungsgemäß bevorzugte Mund- und Zahnpflege- und -reinigungsmittel sind dadurch gekennzeichnet, daß sie - bezogen auf ihr Gewicht - 0,025 - 4,5 Gew.-%, vorzugsweise 0,05 - 4 Gew.-%, besonders bevorzugt 0,1 - 3 Gew.-%, außerordentlich bevorzugt 0,15 - 2 Gew.-%, weiter bevorzugt 0,25 bis 1,5 Gew.-% und insbesondere 0,5 bis 1 Gew.-% Verbindung(en) der Formel (PP-Ia) 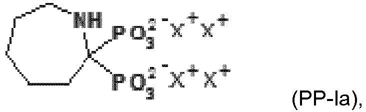 worin,
X⁺ unabhängig voneinander für H⁺, Li⁺, Na⁺, K⁺, NH₄⁺, ½Mg²⁺, ½Ca²⁺, ½Mn²⁺, ½Zn²⁺, ⅓Al³⁺, ¼Zr⁴⁺ steht, enthalten.

Die Verbindung(en) der Formel (PP-I) bewirken zusammen mit dem/den erfindungsgemäßen Coopolymer(en) die weiter verbesserte Entfernung von Oberflächenanschmutzungen und verhindern die Ausbildung von Zahnstein. Besonders effektive Verbindungen der Formel (PP-I) sind solche, in denen R¹ für Wasserstoff steht. Bevorzugte Verbindungen der Formel (PP-I) sind der Tabelle auf den Seiten 11 bis 13 des Prioritätsdokuments zu entnehmen.

Für die Verbindungen der Formel (PP-I) allgemein sowie für die in der Tabelle auf den Seiten 11 bis 13 des Prioritätsdokuments genannten 100 Verbindungen speziell ist es bevorzugt, wenn die 4 Kationen wie folgt definiert sind: 2 Na⁺ 2 H⁺·. Äußerst bevorzugte Verbindungen der Formel (PP-I) sind dadurch gekennzeichnet, daß die Reste R² und R³ eine cyclische Gruppe mit 4 bis 8 Kohlenstoffatomen und gegebenenfalls einem Heteroatom, ausgewählt aus der Gruppe von N, O und S, bilden. Ganz besonders bevorzugte Vertreter dieser Ausführungsform sind in der Tabelle auf den Seiten 14 und 15 des Prioritätsdokuments aufgeführt: Auch für die dort genannten 32 Verbindungen ist es bevorzugt, wenn die 4 Kationen wie folgt definiert sind: 2 Na⁺ 2 H⁺·

Eine ganz besonders bevorzugt einzusetzende Verbindung der Formel (PP-I) ist die Azacycloheptan-Diphosphonsäure. Äußerst bevorzugte erfindungsgemäße Mund- und Zahnpflege- und -reinigungsmittel sind daher dadurch gekennzeichnet, daß sie - bezogen auf ihr Gewicht - 0,025 - 4,5 Gew.-%, vorzugsweise 0,05 - 4 Gew.-%, besonders bevorzugt 0,1 - 3 Gew.-%, außerordentlich bevorzugt 0,15 - 2 Gew.-%, weiter bevorzugt 0,25 bis 1,5 Gew.-% und insbesondere 0,5 bis 1 Gew.-% Verbindung(en) der Formel (PP-la) 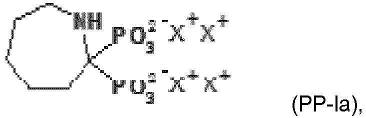 worin,
X⁺ unabhängig voneinander für H⁺, Li⁺, Na⁺, K⁺, NH₄⁺, ½Mg²⁺, ½Ca²⁺, ½Mn²⁺, ½Zn²⁺, ⅓Al³⁺, ¼ Zr⁴⁺ steht, enthalten.

Auch hier ist es bevorzugt, wenn die 4 Kationen wie folgt definiert sind: 2 Na⁺ 2 H⁺·, so daß äußerst bevorzugte erfindungsgemäße Mund und Zahnpflege- und -reinigungsmittel dadurch gekennzeichnet sind, daß sie - bezogen auf ihr Gewicht - 0,025 - 4,5 Gew.-%, vorzugsweise 0,05 - 4 Gew.-%, besonders bevorzugt 0,1 - 3 Gew.-%, außerordentlich bevorzugt 0,15 - 2 Gew.-%, weiter bevorzugt 0,25 bis 1,5 Gew.-% und insbesondere 0,5 bis 1 Gew.-% Dinatrium Azacycloheptan-Diphosphonat enthalten.

Als einen bevorzugten Inhaltsstoff können die erfindungsgemäßen Mittel zusätzlich zu dem/den Copolymer(en) mindestens ein Isopropylmethylphenol enthalten. Bevorzugte erfindungsgemäße Mittel enthalten - bezogen auf ihr Gewicht - 0,001 bis 5 Gew.-% mindestens eines Isopropylmethylphenols.

Besonders bevorzugte Isopropylmethylphenole sind solche der Formeln (I) bzw. (II):

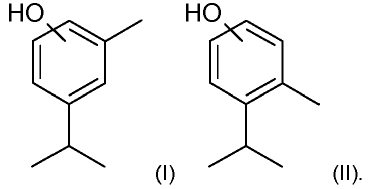

Besonders bevorzugte Vertreter der Formeln (I) bzw, (II) sind

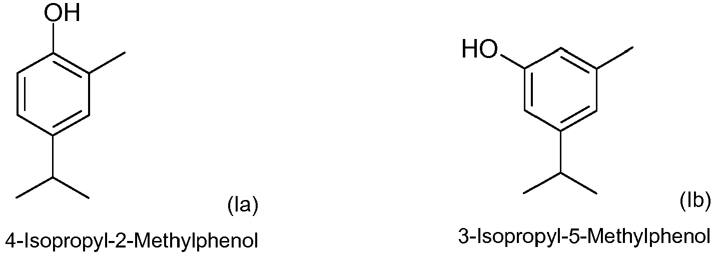

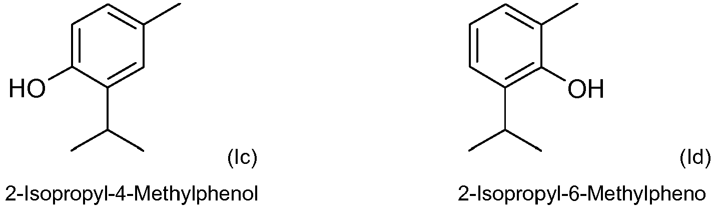

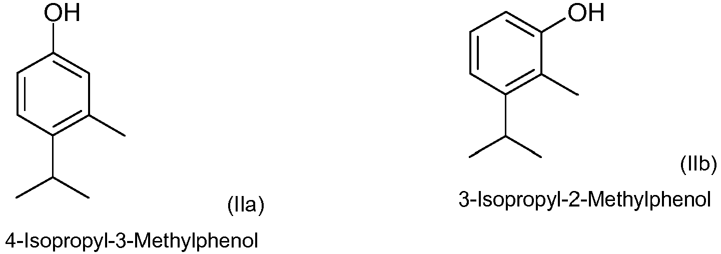

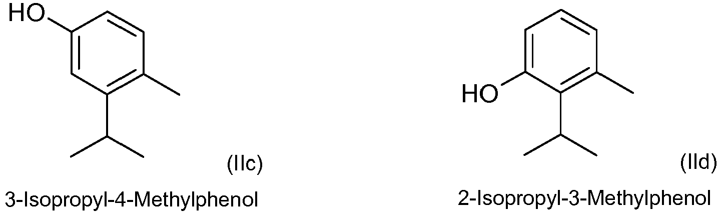

Bevorzugte erfindungsgemäße Mund- und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% (einer) Verbindung(en) aus der Gruppe 4-Isopropyl-2-Methylphenol, 3-Isopropyl-5-Methylphenol, 2-Isopropyl-4-Methylphenol, 2-Isopropyl-6-Methylphenol, 4-Isopropyl-3-Methylphenol, 3-Isopropyl-2-Methylphenol, 3-Isopropyl-4-Methylphenol, 2-Isopropyl-3-Methylphenol enthalten.

Eine bevorzugte Verbindung, die erfindungsgemäß eingesetzt werden kann, ist das 2-Isopropyl-6-Methylphenol der Formel (Id). Diese Verbindung wird auch als Thymol bezeichnet.

Thymol ist ein Monoterpen und neben seinem Isomer Carvacrol ein Bestandteil der ätherischen Öle aus Ajowan, Thymian, Oregano und dem Bohnenkraut. Thymol zeichnet sich durch eine starke desinfizierende fungizide und bakterizide Wirkung aus und wird wegen seines angenehmen Geschmacks in Mundwässern und Zahnpasta eingesetzt.

Bevorzugte erfindungsgemäße Mund- und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Thymol enthalten. Eine besonders bevorzugte Verbindung, die erfindungsgemäß eingesetzt werden kann, ist das 4-Isopropyl-3-Methylphenol der Formel (IIa). Diese Verbindung wird auch als p-Thymol bezeichnet. Bevorzugte erfindungsgemäße Mund- und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% 4-Isopropyl-3-Methylphenol (p-Thymol) enthalten.

Die erfindungsgemäß einzusetzenden Isopropymethylphenole können als einzelne Verbindung oder gemeinsam mit anderen Vertretern aus der Gruppe eingesetzt werden. Im Falle des Einsatzes von 4-Isopropyl-3-Methylphenol (p-Thymol) hat es sich als bevorzugt herausgestellt, die Verbindung als einzigen Vertreter aus der Gruppe der Isopropyl-Methylphenole einzusetzen. In anderen Worten sind besonders bevorzugte erfindungsgemäße Mund- und Zahnpflege- und -reinigungsmittel dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% 4-Isopropyl-3-Methylphenol (p-Thymol) und keine weiteren Isopropyl-methylphenole enthalten.

Es hat sich gezeigt, daß die Wirkung der erfindungsgemäßen Kombination um einen weiteren Effekt gegen Halitosis und insbesondere gegen Gingivitis und Parodontitis ergänzt werden kann, wenn Zinksalze eingesetzt werden.

Erfindungsgemäß bevorzugte Mittel enthalten daher mindestens ein Zinksalz, vorzugsweise in Mengen von 0,001 bis 5 Gew.-%, bezogen auf das gesamte Mittel wobei besonders bevorzugte Mund- und Zahnpflege- und -reinigungsmittel dadurch gekennzeichnet sind, daß sie 0,001 bis 5 Gew.-%, vorzugsweise 0,02 bis 2,5 Gew.-%, besonders bevorzugt 0,03 bis 2,0 Gew.-%, weiter bevorzugt 0,04 bis 1,5 Gew.-%, noch weiter bevorzugt 0,05 bis 1,0 Gew.-%, noch weiter bevorzugt 0,06 bis 0,5 Gew.-% und insbesondere 0,07 bis 0,25 Gew.-% mindestens eines Zinksalzes enthalten.

Hierbei gilt - wie immer, wenn in der vorliegenden Schrift keine anderen Angaben gemacht werden - daß sich die Mengenangaben auf das gesamte Mittel, d.h. das fertige Mund und Zahnpflege- und -reinigungsmittel, beziehen.

Erfindungsgemäß können sowohl anorganische als auch organische Salze des Zinks eingesetzt werden. Neben den nicht löslichen anorganischen Zinksalzen, also Salzen, welche eine Löslichkeit unterhalb 100 mg/L (20°C), vorzugsweise unterhalb 10 mg/L (20°C), insbesondere keine Löslichkeit (20°C) aufweisen (Bsp.: Zinkoxid), sind im Rahmen der vorliegenden Anmeldung die löslichen anorganischen Zinksalze, das heißt Salze, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen, bevorzugter Bestandteil erfindungsgemäßer Mittel. Zu den bevorzugten löslichen anorganischen Salzen zählen das Zinkbromid, das Zinkchlorid, das Zinkiodid, das Zinknitrat und das Zinksulfat.

Erfindungsgemäß besonders bevorzugte Mittel enthalten dabei mindestens ein Zinksalz aus der nachstehenden Tabelle:

| Zinksalz | Löslichkeit |
|---|---|
| Zinkacetat-Dihydrat | 430 g/l (20°C) |
| Zinkacetylacetonat | 4 g/l (20°C) |
| Zinkbromid | 820 g/l (25°C) |
| Zinkchlorid | 4320 g/l (25°C) |
| Zinkgluconat | 100 g/l (20°C) |
| Zinkhydroxycarbonat | Fast unlöslich (20°C) |
| Zinkiodid | 4500 g/l (20°C) |
| Zinknitrat Hexahydrat | 1843 g/l (20°C) |
| Zinknitrat-Tetrahydrat | Leicht löslich (20°C) |
| Zinkoxid | Unlöslich |
| Zinkstearat | 0,9 mg/l (20°C) |
| Zinksulfat-Heptahydat | 960 g/l (20°C) |
| Zinksulfat-Monohydrat | ∼350 g/l (20°C) |

Das Spektrum der erfindungsgemäß bevorzugten Zinksalze organischer Säuren, vorzugsweise organischer Carbonsäuren, reicht von Salzen die in Wasser nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinlaureat, das Zinkoleat, das Zinkoxalat, das Zinktartrat und das Zinkstearat, zu der Gruppe der löslichen organischen Zinksalze gehören beispielsweise das Zinkacetat, das Zinkacetylacetonat, das Zinkbenzoat, das Zinkformiat, das Zinklactat, das Zinkgluconat, das Zinkvalerat sowie das Zinksalz der p-Toluolsulfonsäure.

Es hat sich gezeigt, daß Zinksulfat ein besonders bevorzugt einzusetzendes Zinksalz ist. Zinksulfat bildet mehrere Hydrate. Das Heptahydrat bildet sich aus gesättigter wäßriger Lösung in Form farbloser, glasglänzender, säulenförmiger, rhombischer Kristalle. Oberhalb 39°C erfolgt Umwandlung zu ZnSO₄ · 6H₂O, und bei 70°C liegt nur noch ZnSO₄ · H₂O vor; das letzte H₂O-Molekül entweicht bei 240°C. Interessanterweise steigt die erfindungsgemäße Wirkung bei den Zinksulfaten mit deren Kristallwassergehalt, so daß das Heptahydrat eine besonders bevorzugte Verbindung ist. Erfindungsgemäß bevorzugte Mund- und Zahnpflege- und -reinigungsmittel enthalten daher Zinksulfat, vorzugsweise Zinksulfat-Heptahydrat, wobei bevorzugte erfindungsgemäße Mund und Zahnpflege- und -reinigungsmittel dadurch gekennzeichnet sind, daß sie - bezogen auf ihr Gewicht - 0,001 bis 5 Gew.-%, vorzugsweise 0,02 bis 2,5 Gew.-%, besonders bevorzugt 0,03 bis 2,0 Gew.-%, weiter bevorzugt 0,04 bis 1,5 Gew.-%, noch weiter bevorzugt 0,05 bis 1,0 Gew.-%, noch weiter bevorzugt 0,06 bis 0,5 Gew.-% und insbesondere 0,07 bis 0,25 Gew.-% mindestens eines Zinksalzes, vorzugsweise Zinksulfat-Heptahydrat, enthält.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind erfindungsgemäße Mund- und Zahnpflege- und -reinigungsmittel bevorzugt, die - bezogen auf ihr Gewicht- 10 bis 50 Gew.-%, vorzugsweise 12,5 bis 45 Gew.-%, besonders bevorzugt 15 bis 40 Gew.-%, weiter bevorzugt 17,5 bis 35 Gew.-% und insbesondere 20 bis 29 Gew.-% mehrwertige(n) Alkohol(e) aus der Gruppe Sorbit und/oder Glycerin und/oder 1,2-Propylenglycol oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Die erfindungsgemäßen Zusammensetzungen können zusätzlich mindestens ein Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, dass sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders geeignet sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, besonders geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident® 8 (Evonik) und Sorbosil® AC 39 (PQ Corporation). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pas aufweisen.

Zahnpasten, die eine deutlich höhere Viskosität von mehr als 100 Pas (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 - 250 m²/g zu, z.B. das Handelsprodukt Sident® 22S.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben sich als besonders effizient bei der Lösung der erfindungsgemäßen Aufgabe erwiesen.

Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und -reinigungsmittel enthalten 2,5 bis 19,5 Gew.-%, vorzugsweise 5 bis 19 Gew.-%, besonders bevorzugt 7,5 bis 18,5 Gew.-%, weiter bevorzugt 8,0 bis 18 Gew.-% und insbesondere 10,0 bis 17,5 Gew.-% Fällungskieselsäure(n) mit einer spezifischen Oberfläche nach ISO 5794-1, Anhang D von ≤ 60 m²/g, vorzugsweise von ≤ 52,5 m²/g, weiter bevorzugt von ≤ 49 m²/g und insbesondere von ≤ 47 m²/g.

Als weiteren wesentlichen Inhaltsstoff können die erfindungsgemäßen Mund- und Zahnpflege- und -reinigungsmittel mindestens ein anionisches Tensid enthalten.

Geeignete anionische Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe.

Weitere typische Beispiele für anionische Tenside sind Seifen, Alkylbenzolsulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid-(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)-phosphate. Sofern die anionischen Tenside Polyglycoletherketten enthalten, können diese eine konventionelle, vorzugsweise jedoch eine eingeengte Homologenverteilung aufweisen. Erfindungsgemäß bevorzugte Mund- und Zahnpflege- und -reinigungsmittel sind dadurch gekennzeichnet, daß sie 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,25 bis 2,2 Gew.-% anionische(s) Tensid(e) enthalten.

Als weiteren Inhaltsstoff können die erfindungsgemäßen Mund- und Zahnpflege- und - reinigungsmittel mindestens ein amphoteres Tensid enthalten.

Amphotere Tenside werden in ampholytische Tenside und zwitterionische Tenside unterteilt. Unter ampholytischen Tensiden werden solche oberflächenaktiven Verbindungen verstanden, die sowohl saure als auch basische hydrophile Gruppen besitzen und sich also je nach Bedingung sauer oder basisch verhalten.

Geeignete ampholytische Tenside enthalten beispielsweise außer einer C₈ - C₂₄ - Alkyl- oder -Acylgruppe mindestens eine freie Aminogruppe (basische hydrophile Gruppe) und mindestens eine - COOH- oder -SO₃H-Gruppe (saure hydrophile Gruppe) und sind zur Ausbildung innerer Salze befähigt. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, dass sie amphotere(s) Tensid(e) aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat, C₁₂ -C₁₈-Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispielsweise Kokosacylaminopropyl-dimethylammoniumglycinat, 2-Alkyl-3-carboxymethyl-3-hydroxyethylimidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen enthalten.

Weiterhin ebenfalls bevorzugte Mund und Zahnpflege- und -reinigungsmittel enthalten als amphotere Tenside Betaine der Formel (Bet-I) 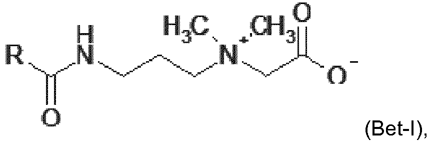 in der R für einen geradkettigen oder verzweigten, gesättigten oder ein- bzw. mehrfach ungesättigten Alkyl- oder Alkenylrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

In einer weiteren besonders bevorzugten Ausführungsform ist ein erfindungsgemäßes Mund- und Zahnpflege- und -reinigungsmittel daher dadurch gekennzeichnet, dass es, bezogen auf sein Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% amphotere(s) Tensid(e), vorzugsweise Cocamidopropylbetain enthält.

Mund- und Zahnpflege- und reinigungsmittel, insbesondere Zahnpasten, können zusätzlich auch weitere Substanzen enthalten, die gegen Plaque und/oder Zahnstein wirksam sind. Hierzu eignen sich beispielsweise antimikrobielle Stoffe und Konservierungsmittel (Plaque) oder Chelatbildner (Zahnstein).

Erfindungsgemäß bevorzugte Mund- und Zahnpflege- und -reinigungsmittel sind dadurch gekennzeichnet, dass sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.
Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Die erfindungsgemäßen Zahnpflegemittel können z.B. durch Zusatz von Aromaölen und Süßungsmitteln in ihren organoleptischen Eigenschaften verbessert werden.
Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.
Erfindungsgemäße Mittel können als Zahnpasten oder Zahncremes formuliert werden.

## Patentansprüche

1. Mund- und Zahnpflege- und -reinigungsmittel, enthaltend - bezogen auf sein Gewicht -
a) 0,1 bis 5 Gew.-% Copolymer(e), umfassend mindestens eine Struktureinheit der Formel (A1) und/oder mindestens eine Struktureinheit der Formel (A2) 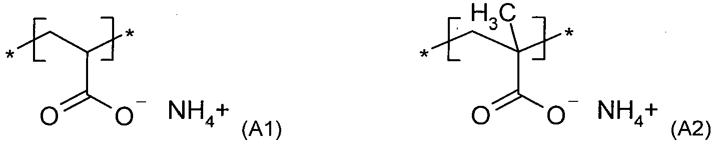
mit der Maßgabe, daß es - bezogen auf sein Gewicht - 0,15 - 4 Gew.-%, vorzugsweise 0,2 - 3 Gew.-%, besonders bevorzugt 0,25 - 2,75 Gew.-%, außerordentlich bevorzugt 0,5 - 2,5 Gew.-% und insbesondere 1 bis 2 Gew.-% Copolymer(e) aus der Gruppe der Copolymere, die jeweils ausschließlich aus folgenden Monomeren aufgebaut sind:
- Ammoniumacrylat und Acrylsäure;
- Ammoniumacrylat und Methacrylsäure;
- Ammoniumacrylat und Acrylsäureethylester;
- Ammoniumacrylat und Methacrylsäureethylester;
- Ammoniummethacrylat und Acrylsäure;
- Ammoniummethacrylat und Methacrylsäure;
- Ammoniummethacrylat und Acrylsäureethylester;
- Ammoniummethacrylat und Methacrylsäureethylester;
- Ammoniumacrylat und Ammoniummethacrylat und Acrylsäure;
- Ammoniumacrylat und Ammoniummethacrylat und Methacrylsäure;
- Ammoniumacrylat und Ammoniummethacrylat und Acrylsäureethylester;
- Ammoniumacrylat und Ammoniummethacrylat und Methacrylsäureethylester;
- Ammoniumacrylat und Acrylsäure und Methacrylsäure;
- Ammoniumacrylat und Acrylsäure und Acrylsäureethylester;
- Ammoniumacrylat und Acrylsäure und Methacrylsäureethylester;
- Ammoniumacrylat und Methacrylsäure und Acrylsäureethylester;
- Ammoniumacrylat und Methacrylsäure und Methacrylsäureethylester;
- Ammoniumacrylat und Acrylsäureethylester und Methacrylsäureethylester;
- Ammoniummethacrylat und Acrylsäure und Methacrylsäure;
- Ammoniummethacrylat und Acrylsäure und Acrylsäureethylester;
- Ammoniummethacrylat und Acrylsäure und Methacrylsäureethylester;
- Ammoniummethacrylat und Methacrylsäure und Acrylsäureethylester;
- Ammoniummethacrylat und Methacrylsäure und Methacrylsäureethylester;
- Ammoniummethacrylat und Acrylsäureethylester und Methacrylsäureethylester;
- Ammoniumacrylat und Ammoniummethacrylat und Acrylsäure und Methacrylsäure;
- Ammoniumacrylat und Ammoniummethacrylat und Acrylsäure und Acrylsäureethylester;
- Ammoniumacrylat und Ammoniummethacrylat und Acrylsäure und Methacrylsäureethylester;
- Methacrylsäuremethylester;
- Ammoniumacrylat und Ammoniummethacrylat und Methacrylsäure und Acrylsäureethylester;
- Ammoniumacrylat und Ammoniummethacrylat und Methacrylsäure und Methacrylsäureethylester;
- Ammoniumacrylat und Ammoniummethacrylat und Acrylsäureethylester und Methacrylsäureethylester;
enthält.

2. Mund- und Zahnpflege- und reinigungsmittel nach Anspruch 1, **dadurch gekennzeichnet, daß** das/die Copolymer(e), umfassend mindestens eine Struktureinheit der Formel (A1) und mindestens eine Struktureinheit der Formel (A2), Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

3. Mund- und Zahnpflege- und reinigungsmittel nach einem der Ansprüche 1 oder 2, **dadurch gekennzeichnet, daß** es mindestens einen weiteren Inhaltsstoff aus der Gruppe
- der blauen Farbstoffe und/oder
- der Verbindungen der Formel (PP-I) 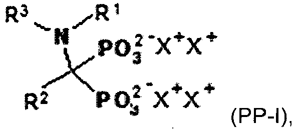 worin
R¹ für Wasserstoff oder eine lineare oder verzweigte Alkylgruppe mit 1 bis 6 Kohlenstoffatomen steht,
R² und R³, jeweils unabhängig voneinander, für eine lineare oder verzweigte Alkylgruppe mit 1 bis 6 Kohlenstoffatomen stehen oder R² und R³ eine cyclische Gruppe mit 4 bis 8 Kohlenstoffatomen und gegebenenfalls einem Heteroatom, ausgewählt aus der Gruppe von N, O und S, bilden, und
X⁺ unabhängig voneinander für H⁺, Li⁺, Na⁺, K⁺, NH₄⁺, ½Mg²⁺, ½Ca²⁺, ½Mn²⁺, ½ Zn²⁺, ⅓Al³⁺, ¼Zr4⁺ steht und/oder
- der Isopropylmethylphenole und/oder
- Pentanatriumtriphosphat und/oder
- der Zinksalze
enthält.

4. Mund- und Zahnpflege- und reinigungsmittel nach einem der Ansprüche 1 bis 3, **dadurch gekennzeichnet, daß** es bezogen auf sein Gewicht 0,0002 bis 0,5 Gew.-%, vorzugsweise 0,0005 bis 0,25 Gew.-%, besonders bevorzugt 0,001 bis 0,1 Gew.-% und insbesondere 0,002 bis 0,05 Gew.-% *4-[4,4'-Bis-diethylamino-α-hydroxy-benzhydryl]-6-hydroxy-benzol-1,3-disulfonsäure* (Patentblau V) 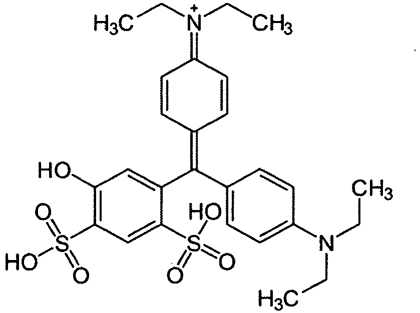 enthält.

5. Mund- und Zahnpflege- und reinigungsmittel nach einem der Ansprüche 1 bis 4, **dadurch gekennzeichnet, daß** es bezogen auf sein Gewicht 0,0002 bis 0,5 Gew.-%, vorzugsweise 0,0005 bis 0,25 Gew.-%, besonders bevorzugt 0,001 bis 0,1 Gew.-% und insbesondere 0,002 bis 0,05 Gew.-% Indigotin-5,5'-disulfonsäure Dinatriumsalz 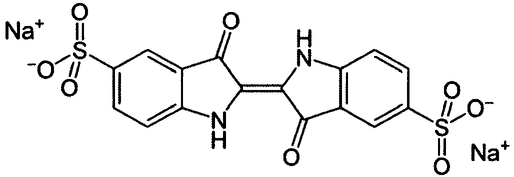 enthält.

6. Mund- und Zahnpflege- und reinigungsmittel nach einem der Ansprüche 1 bis 5, **dadurch gekennzeichnet, daß** es - bezogen auf sein Gewicht - 0,0002 bis 0,5 Gew.-%, vorzugsweise 0,0005 bis 0,25 Gew.-%, besonders bevorzugt 0,001 bis 0,1 Gew.-% und insbesondere 0,001 bis 0,05 Gew.-% mindestens eines blauen Farbstoffes, bevorzugt Benzenemethanaminium,N-ethyl-N-[4-[[4-[ethyl[(3-sulfophenyl)methyl]amino]phenyl](2-sulfophenyl)methylene]-2,5-cyclohexadien-1-ylidene]-3-sulfo-,inner salt, sodium salt (Brillantblau FCF) 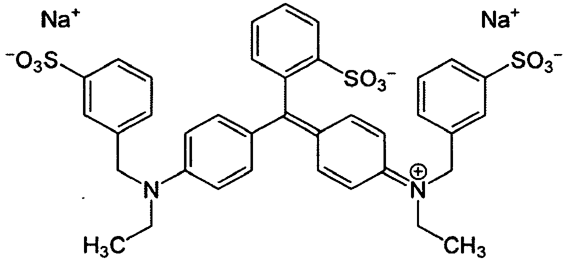 enthält.

7. Mund- und Zahnpflege- und -reinigungsmittel nach einem der Ansprüche 1 bis 6, **dadurch gekennzeichnet, daß** es - bezogen auf sein Gewicht - 0,025 - 4,5 Gew.-%, vorzugsweise 0,05 - 4 Gew.-%, besonders bevorzugt 0,1 - 3 Gew.-%, außerordentlich bevorzugt 0,15 - 2 Gew.-%, weiter bevorzugt 0,25 bis 1,5 Gew.-% und insbesondere 0,5 bis 1 Gew.-% Verbindung(en) der Formel (PP-Ia) 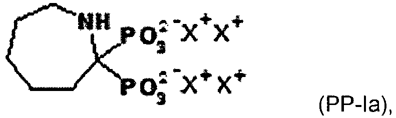 worin,
X⁺ unabhängig voneinander für H⁺, Li⁺, Na⁺, K⁺, NH₄⁺, ½Mg²⁺, ½Ca²⁺, ½Mn²⁺, ½Zn²⁺, ⅓ Al³⁺, ¼Zr⁴⁺ steht, enthält.

8. Mund und Zahnpflege- und -reinigungsmittel nach einem der Ansprüche 1 bis 7, **dadurch gekennzeichnet, daß** es - bezogen auf sein Gewicht - 0,025 - 4,5 Gew.-%, vorzugsweise 0,05 - 4 Gew.-%, besonders bevorzugt 0,1 - 3 Gew.-%, außerordentlich bevorzugt 0,15 - 2 Gew.-%, weiter bevorzugt 0,25 bis 1,5 Gew.-% und insbesondere 0,5 bis 1 Gew.-% Dinatrium Azacycloheptan-Diphosphonat enthält.

9. Mund und Zahnpflege- und -reinigungsmittel nach einem der Ansprüche 1 bis 8, **dadurch gekennzeichnet, daß** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% (einer) Verbindung(en) aus der Gruppe
- 4-Isopropyl-2-Methylphenol
- 3-Isopropyl-5-Methylphenol
- 2-Isopropyl-4-Methylphenol
- 2-Isopropyl-6-Methylphenol
- 4-Isopropyl-3-Methylphenol
- 3-Isopropyl-2-Methylphenol
- 3-Isopropyl-4-Methylphenol
- 2-Isopropyl-3-Methylphenol
enthält.

10. Mund und Zahnpflege- und -reinigungsmittel nach einem der Ansprüche 1 bis 9, **dadurch gekennzeichnet, daß** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Thymol enthält.

11. Mund und Zahnpflege- und -reinigungsmittel nach einem der Ansprüche 1 bis 10, **dadurch gekennzeichnet, daß** es - bezogen auf sein Gewicht - 0,001 bis 5 Gew.-%, vorzugsweise 0,02 bis 2,5 Gew.-%, besonders bevorzugt 0,03 bis 2,0 Gew.-%, weiter bevorzugt 0,04 bis 1,5 Gew.-%, noch weiter bevorzugt 0,05 bis 1,0 Gew.-%, noch weiter bevorzugt 0,06 bis 0,5 Gew.-% und insbesondere 0,07 bis 0,25 Gew.-% mindestens eines Zinksalzes, vorzugsweise Zinksulfat-Heptahydrat, enthält.

## Claims

1. An oral and dental care and cleaning agent, containing, based on the weight thereof,
a) from 0.1 to 5 wt.% copolymer(s), comprising at least one structural unit of formula (A1) and/or at least one structural unit of formula (A2) 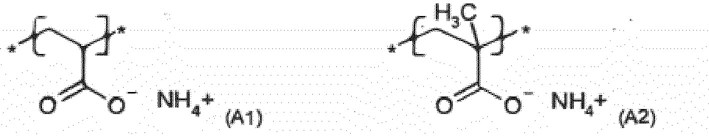
with the proviso that said agent contains, based on the weight thereof, from 0.15 to 4 wt.%, preferably from 0.2 to 3 wt.%, particularly preferably from 0.25 to 2.75 wt.%, extremely preferably from 0.5 to 2.5 wt.%, and in particular from 1 to 2 wt.% copolymer(s) from the group of copolymers each composed exclusively of the following monomers:
- ammonium acrylate and acrylic acid;
- ammonium acrylate and methacrylic acid;
- ammonium acrylate and acrylic acid ethyl ester;
- ammonium acrylate and methacrylic acid ethyl ester;
- ammonium methacrylate and acrylic acid;
- ammonium methacrylate and methacrylic acid;
- ammonium methacrylate and acrylic acid ethyl ester;
- ammonium methacrylate and methacrylic acid ethyl ester;
- ammonium acrylate and ammonium methacrylate and acrylic acid;
- ammonium acrylate and ammonium methacrylate and methacrylic acid;
- ammonium acrylate and ammonium methacrylate and acrylic acid ethyl ester;
- ammonium acrylate and ammonium methacrylate and methacrylic acid ethyl ester;
- ammonium acrylate and acrylic acid and methacrylic acid;
- ammonium acrylate and acrylic acid and acrylic acid ethyl ester;
- ammonium acrylate and acrylic acid and methacrylic acid ethyl ester;
- ammonium acrylate and methacrylic acid and acrylic acid ethyl ester;
- ammonium acrylate and methacrylic acid and methacrylic acid ethyl ester;
- ammonium acrylate and acrylic acid ethyl ester and methacrylic acid ethyl ester;
- ammonium methacrylate and acrylic acid and methacrylic acid;
- ammonium methacrylate and acrylic acid and acrylic acid ethyl ester;
- ammonium methacrylate and acrylic acid and methacrylic acid ethyl ester;
- ammonium methacrylate and methacrylic acid and acrylic acid ethyl ester;
- ammonium methacrylate and methacrylic acid and methacrylic acid ethyl ester;
- ammonium methacrylate and acrylic acid ethyl ester and methacrylic acid ethyl ester;
- ammonium acrylate and ammonium methacrylate and acrylic acid and methacrylic acid;
- ammonium acrylate and ammonium methacrylate and acrylic acid and acrylic acid ethyl ester;
- ammonium acrylate and ammonium methacrylate and acrylic acid and methacrylic acid ethyl ester;
- methacrylic acid methyl ester;
- ammonium acrylate and ammonium methacrylate and methacrylic acid and acrylic acid ethyl ester;
- ammonium acrylate and ammonium methacrylate and methacrylic acid and
- methacrylic acid ethyl ester;
- ammonium acrylate and ammonium methacrylate and acrylic acid ethyl ester and methacrylic acid ethyl ester.

2. The oral and dental care and cleaning agent according to claim 1, **characterized in that** the copolymer(s) comprising at least one structural unit of formula (A1) and at least one structural unit of formula (A2) has/have molar masses of from 500,000 to 5,000,000 daltons, preferably from 600,000 to 4,000,000 daltons, more preferably from 700,000 to 3,000,000 daltons, even more preferably from 800,000 to 2,000,000 daltons, and in particular from 900,000 to 1,500,000 daltons.

3. The oral and dental care and cleaning agent according to one of claims 1 or 2, **characterized in that** it contains at least one further ingredient from the group of
- blue dyes and/or
- compounds of formula (PP-I) 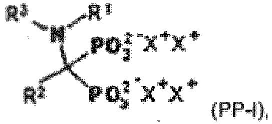 in which
R¹ represents hydrogen or a linear or branched alkyl group having 1 to 6 carbon atoms,
R² and R³, independently of one another, each represent a linear or branched alkyl group having 1 to 6 carbon atoms or R² and R³ form a cyclic group having 4 to 8 carbon atoms and optionally a heteroatom selected from the group of N, O and S, and
X⁺ independently represents H⁺, Li⁺, Na⁺, K⁺, NH₄⁺, ½Mg²⁺, ½Ca²⁺, ½Mn²⁺, ½Zn²⁺, ⅓ Al³⁺, ¼Zr⁴⁺ and/or
- isopropyl methylphenols and/or
- pentasodium triphosphate and/or
- zinc salts.

4. The oral and dental care and cleaning agent according to one of claims 1 to 3, **characterized in that** it contains, based on the weight thereof, from 0.0002 to 0.5 wt.%, preferably from 0.0005 to 0.25 % wt.%, particularly preferably from 0.001 to 0.1 wt.%, and in particular from 0.002 to 0.05 wt.% *4-[4,4'-bis-diethylamino-α-hydroxy-benzhydryl]-6-hydroxy-benzol-1,3-disulfonic acid* (Patent Blue V)

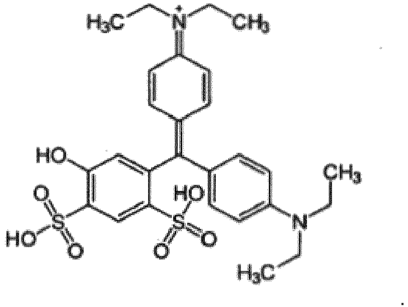

5. The oral and dental care and cleaning agent according to one of claims 1 to 4, **characterized in that** it contains, based on the weight thereof, from 0.0002 to 0.5 wt.%, preferably from 0.0005 to 0.25 wt.%, particularly preferably from 0.001 to 0.1 wt.% and in particular from 0.002 to 0.05 wt.% indigotine-5,5'-disulfonic acid disodium salt

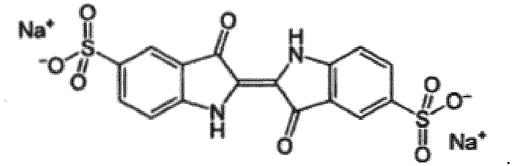

6. The oral and dental care and cleaning agent according to one of claims 1 to 5, **characterized in that** it contains, based on the weight thereof, from 0.0002 to 0.5 wt.%, preferably from 0.0005 to 0.25 wt.%, particularly preferably from 0.001 to 0.1 wt.% and in particular from 0.001 to 0.05 wt.% of at least one blue dye, preferably benzenemethanaminium,N-ethyl-N-[4-[[4-[ethyl[(3-sulfophenyl)methyl]amino]phenyl](2-sulfophenyl)methylene]-2,5-cyclohexadiene-1-ylidene]-3-sulfo-, inner salt, sodium salt (Brilliant Blue FCF)

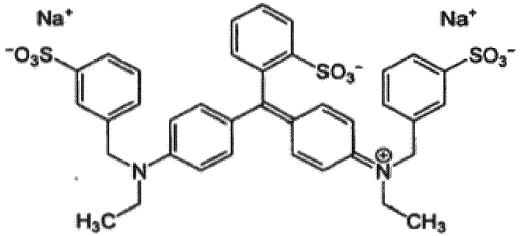

7. The oral and dental care and cleaning agent according to one of claims 1 to 6, **characterized in that** it contains, based on the weight thereof, from 0.025 to 4.5 wt.%, preferably from 0.05 to 4 wt.%, particularly preferably from 0.1 to 3 wt.%, extremely preferably from 0.15 to 2 wt.%, more preferably from 0.25 to 1.5 wt.% and in particular from 0.5 to 1 wt.% compound(s) of formula (PP-Ia) 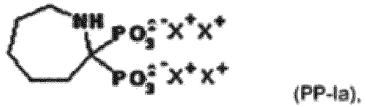 in which
X⁺ independently represents H⁺, Li⁺, Na⁺, K⁺, NH₄⁺, ½Mg²⁺, ½Ca²⁺, ½Mn²⁺, ½Zn²⁺, ⅓ Al³⁺, ¼Zr⁴⁺.

8. The oral and dental care and cleaning agent according to one of claims 1 to 7, **characterized in that** it contains, based on the weight thereof, from 0.025 to 4.5 wt.%, preferably from 0.05 to 4 wt.%, particularly preferably from 0.1 to 3 wt.%, extremely preferably from 0.15 to 2 wt.%, more preferably from 0.25 to 1.5 wt.% and in particular from 0.5 to 1 wt.% disodium azacycloheptane diphosphonate.

9. The oral and dental care and cleaning agent according to one of claims 1 to 8, **characterized in that** it contains, based on the weight thereof, from 0.002 to 4 wt.%, preferably from 0.003 to 3 wt.%, particularly preferably from 0.004 to 2 wt.%, extremely preferably from 0.005 to 1 wt.%, more preferably from 0.01 to 0.5 wt.% and in particular from 0.05 to 0.2 wt.% (of) compound(s) from the group
- 4-isopropyl-2-methylphenol
- 3-isopropyl-5-methylphenol
- 2-isopropyl-4-methylphenol
- 2-isopropyl-6-methylphenol
- 4-isopropyl-3-methylphenol
- 3-isopropyl-2-methylphenol
- 3-isopropyl-4-methylphenol
- 2-isopropyl-3-methylphenol.

10. The oral and dental care and cleaning agent according to one of claims 1 to 9, **characterized in that** it contains, based on the weight thereof, from 0.002 to 4 wt.%, preferably from 0.003 to 3 wt.%, particularly preferably from 0.004 to 2 wt.%, extremely preferably from 0.005 to 1 wt.%, more preferably from 0.01 to 0.5 wt.% and in particular from 0.05 to 0.2 wt.% thymol.

11. The oral and dental care and cleaning agent according to one of claims 1 to 10, **characterized in that** it contains, based on the weight thereof, from 0.001 to 5 wt.%, preferably from 0.02 to 2.5 wt.%, particularly preferably from 0.03 to 2.0 wt.%, more preferably from 0.04 to 1.5 wt.%, even more preferably from 0.05 to 1.0 wt.%, even further preferably from 0.06 to 0.5 wt.%, and in particular from 0.07 to 0.25 wt.%, of at least one zinc salt, preferably zinc sulfate heptahydrate.

## Revendications

1. Agent de nettoyage et de soins buccaux-dentaires contenant - rapporté à son poids -
a) de 0,1 à 5 % en poids de copolymère(s) comprenant au moins un motif structurel de formule (A1) et/ou au moins un motif structurel de formule (A2) 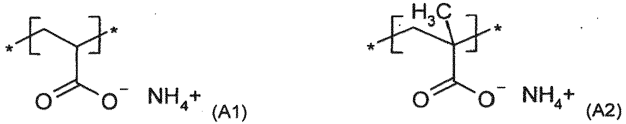
à condition qu'il contienne - rapporté à son poids - de 0,15 à 4 % en poids, de préférence de 0,2 à 3 % en poids, de manière particulièrement préférée de 0,25 à 2,75 % en poids, de manière extrêmement préférée de 0,5 à 2,5 % en poids et en particulier de 1 à 2 % en poids de copolymère(s) issu(s) du groupe des copolymères, qui sont respectivement composés exclusivement des monomères suivants :
- acrylate d'ammonium et acide acrylique ;
- acrylate d'ammonium et acide méthacrylique ;
- acrylate d'ammonium et ester éthylique d'acide acrylique ;
- acrylate d'ammonium et ester éthylique d'acide méthacrylique ;
- méthacrylate d'ammonium et acide acrylique ;
- méthacrylate d'ammonium et acide méthacrylique ;
- méthacrylate d'ammonium et ester éthylique d'acide acrylique ;
- méthacrylate d'ammonium et ester éthylique d'acide méthacrylique ;
- acrylate d'ammonium et méthacrylate d'ammonium et acide acrylique ;
- acrylate d'ammonium et méthacrylate d'ammonium et acide méthacrylique ;
- acrylate d'ammonium et méthacrylate d'ammonium et ester éthylique d'acide acrylique ;
- acrylate d'ammonium et méthacrylate d'ammonium et ester éthylique d'acide méthacrylique ;
- acrylate d'ammonium et acide acrylique et acide méthacrylique ;
- acrylate d'ammonium et acide acrylique et ester éthylique d'acide acrylique ;
- acrylate d'ammonium et acide acrylique et ester éthylique d'acide méthacrylique ;
- acrylate d'ammonium et acide méthacrylique et ester éthylique d'acide acrylique ;
- acrylate d'ammonium et acide méthacrylique et ester éthylique d'acide méthacrylique ;
- acrylate d'ammonium et ester éthylique d'acide acrylique et ester éthylique d'acide méthacrylique ;
- méthacrylate d'ammonium et acide acrylique et acide méthacrylique ;
- méthacrylate d'ammonium et acide acrylique et ester éthylique d'acide acrylique ;
- méthacrylate d'ammonium et acide acrylique et ester éthylique d'acide méthacrylique ;
- méthacrylate d'ammonium et acide méthacrylique et ester éthylique d'acide acrylique ;
- méthacrylate d'ammonium et acide méthacrylique et ester éthylique d'acide méthacrylique ;
- méthacrylate d'ammonium et ester éthylique d'acide acrylique et ester éthylique d'acide méthacrylique ;
- acrylate d'ammonium et méthacrylate d'ammonium et acide acrylique et acide méthacrylique ;
- acrylate d'ammonium et méthacrylate d'ammonium et acide acrylique et ester éthylique d'acide acrylique ;
- acrylate d'ammonium et méthacrylate d'ammonium et acide acrylique et ester éthylique d'acide méthacrylique ;
- ester méthylique d'acide méthacrylique ;
- acrylate d'ammonium et méthacrylate d'ammonium et acide méthacrylique et ester éthylique d'acide acrylique ;
- acrylate d'ammonium et méthacrylate d'ammonium et acide méthacrylique et ester éthylique d'acide méthacrylique ;
- acrylate d'ammonium et méthacrylate d'ammonium et ester éthylique d'acide acrylique et ester éthylique d'acide méthacrylique.

2. Agent de nettoyage et de soins buccaux-dentaires selon la revendication 1, **caractérisé en ce que** le ou les copolymères comprenant au moins un motif structurel de formule (A1) et au moins un motif structurel de formule (A2) ont des masses molaires allant de 500 000 à 5 000 000 daltons, de préférence de 600 000 à 4 000 000 daltons, de manière davantage préférée de 700 000 à 3 000 000, de manière encore davantage préférée de 800 000 à 2 000 000 daltons, et en particulier de 900 000 à 1 500 000 daltons.

3. Agent de nettoyage et de soins buccaux-dentaires selon l'une des revendications 1 ou 2, **caractérisé en ce qu'**il contient au moins un autre ingrédient issu du groupe
- des colorants bleus et/ou
- des composés de formule (PP-I) 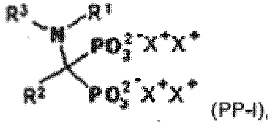 dans laquelle
R¹ représente l'hydrogène ou un groupe alkyle linéaire ou ramifié ayant de 1 à 6 atomes de carbone,
R² et R³ représentent, chacun indépendamment l'un de l'autre, un groupe alkyle linéaire ou ramifié ayant de 1 à 6 atomes de carbone, ou R² et R³ forment un groupe cyclique ayant de 4 à 8 atomes de carbone et éventuellement un hétéroatome, choisi dans le groupe comprenant N, O et S, et
X⁺ représentent indépendamment les uns des autres H⁺, Li⁺, Na⁺, K⁺, NH₄⁺, ½Mg²⁺, ½ Ca²⁺, ½Mn²⁺, ½Zn²⁺, 1/3 Al³⁺, ¼Zr⁴⁺ et/ou
- des isopropylméthylphénols et/ou
- du triphosphate pentasodique et/ou
- des sels de zinc.

4. Agent de nettoyage et de soins buccaux-dentaires selon l'une des revendications 1 à 3, **caractérisé en ce qu'**il contient - rapporté à son poids - de 0,0002 à 0,5 % en poids, de préférence de 0,0005 à 0,25 % en poids, de manière particulièrement préférée de 0,001 à 0,1 % en poids, et en particulier de 0,002 à 0,05 % en poids d'acide 4-[4,4'bis-diéthylamino-α-hydroxy-benzhydryle]-6-hydroxy-benzène-1,3-disulfonique (bleu breveté V)

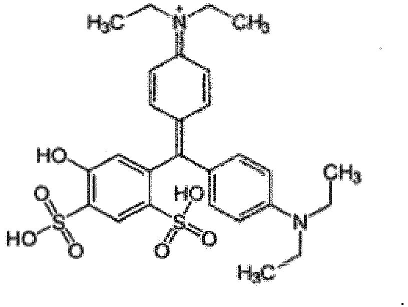

5. Agent de nettoyage et de soins buccaux-dentaires selon l'une des revendications 1 à 4, **caractérisé en ce qu'**il contient, rapporté à son poids, de 0,0002 à 0,5 % en poids, de préférence de 0,0005 à 0,25 % en poids, de manière particulièrement préférée de 0,001 à 0,1 % en poids et en particulier de 0,002 à 0,05 % en poids de sel disodique d'acide indigotine-5,5'-disulfonique

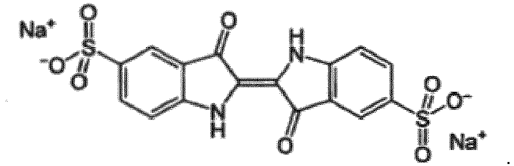

6. Agent de nettoyage et de soins buccaux-dentaires selon l'une des revendications 1 à 5, **caractérisé en ce qu'**il contient - rapporté à son poids - de 0,0002 à 0,5 % en poids, de préférence de 0,0005 à 0,25 % en poids, de manière particulièrement préférée de 0,001 à 0,1 % en poids, et en particulier de 0,001 à 0,05 % en poids d'au moins un colorant bleu, de manière préférée de Benzenemethanaminium,N-ethyl-N-[4-[[4-[ethyl[(3-sulfophenyl)methyl]amino]phenyl](2-sulfophenyl)methylene]-2,5-cyclohexadien-1-ylidene]-3-sulfo-, inner salt, sodium salt (bleu brillant FCF)

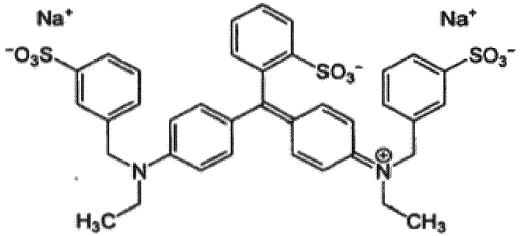

7. Agent de nettoyage et de soins buccaux-dentaires selon l'une des revendications 1 à 6, **caractérisé en ce qu'**il contient - rapporté à son poids - de 0,025 à 4,5 % en poids, de préférence de 0,05 à 4 % en poids, de manière particulièrement préférée de 0,1 à 3 % en poids, de manière extrêmement préférée de 0,15 à 2 % en poids, de manière davantage préférée de 0,25 à 1,5 % en poids et en particulier 0,5 à 1 % en poids de composé(s) de formule (PP-Ia) 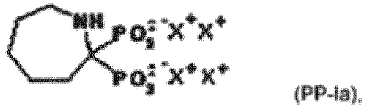 dans laquelle
X⁺ représentent indépendamment les un des autres H⁺, Li⁺, Na⁺, K⁺, NH₄⁺, ½Mg²⁺, ½Ca²⁺, ½ Mn²⁺, ½Zn²⁺, 1/3 Al³⁺, ¼Zr⁴⁺.

8. Agent de nettoyage et de soins buccaux-dentaires selon l'une des revendications 1 à 7, **caractérisé en ce qu'**il contient - rapporté à son poids - de 0,025 à 4,5 % en poids, de préférence de 0,05 à 4 % en poids, de manière particulièrement préférée de 0,1 à 3 % en poids, de manière extrêmement préférée de 0,15 à 2 % en poids, de manière davantage préférée de 0,25 à 1,5 % en poids et en particulier 0,5 à 1 % en poids d'azacycloheptane diphosphonate de disodium.

9. Agent de nettoyage et de soins buccaux-dentaires selon l'une des revendications 1 à 8, **caractérisé en ce qu'**il contient - rapporté à son poids - de 0,002 à 4 % en poids, de préférence de 0,003 à 3 % en poids, de manière particulièrement préférée de 0,004 à 2 % en poids, de manière extrêmement préférée de 0,005 à 1 % en poids, de manière davantage préférée de 0,01 à 0,5 % en poids et en particulier de 0,05 à 0,2 % en poids d'un/de composé(s) issu(s) du groupe
- 4-isopropyl-2-méthylphénol
- 3-isopropyl-5-méthylphénol
- 2-isopropyl-4-méthylphénol
- 2-isopropyl-6-méthylphénol
- 4-isopropyl-3-méthylphénol
- 3-isopropyl-2-méthylphénol
- 3-isopropyl-4-méthylphénol
- 2-isopropyl-3-méthylphénol.

10. Agent de nettoyage et de soins buccaux-dentaires selon l'une des revendications 1 à 9, **caractérisé en ce qu'**il contient - rapporté à son poids - de 0,002 à 4 % en poids, de préférence de 0,003 à 3 % en poids, de manière particulièrement préférée de 0,004 à 2 % en poids, de manière extrêmement préférée de 0,005 à 1 % en poids, de manière davantage préférée de 0,01 à 0,5 % en poids et en particulier 0,05 à 0,2 % en poids de thymol.

11. Agent de nettoyage et de soins buccaux-dentaires selon l'une des revendications 1 à 10, **caractérisé en ce qu'**il contient - rapporté à son poids - de 0,001 à 5 % en poids, de préférence de 0,02 à 2,5 % en poids, de manière particulièrement préférée de 0,03 à 2,0 % en poids, de manière davantage préférée de 0,04 à 1,5 % en poids, de manière encore davantage préférée de 0,05 à 1,0 % en poids, de manière encore davantage préférée de 0,06 à 0,5 % en poids et en particulier de 0,07 à 0,25 % en poids d'au moins un sel de zinc, de préférence l'heptahydrate de sulfate de zinc.
